# EUROPEAN PATENT SPECIFICATION

(11) **EP 0 502 504 B1**
(45) Date of publication and mention of the grant of the patent: **02.07.1997**
(21) Application number: 92103691.9
(22) Date of filing: 04.03.1992
(51) Int. Cl.: C12Q 1/00, G01N 27/404

(54) **A biosensor utilizing enzyme and a method for producing the same**
Enzymbiosensor und Verfahren zur Herstellung desselben
Biocapteur à enzyme et son procédé de fabrication

(30) Priority: 04.03.1991 JP 37259/91; 04.03.1991 JP 37261/91; 02.09.1991 JP 221402/91
(43) Date of publication of application: 09.09.1992
(73) Proprietor: MATSUSHITA ELECTRIC INDUSTRIAL CO., LTD., Kadoma-shi, Osaka 571 (JP)
(72) Inventor: Yoshioka, Toshihiko, Osaka-shi, Osaka (JP); Nankai, Shiro, Hirakata-shi, Osaka (JP)
(74) Representative: Marx, Lothar, Dr.

(56) References cited:
- EP-A- 0 359 831
- EP-A- 0 400 918
- WO-A-91/09139
- PATENT ABSTRACTS OF JAPAN vol. 13, no. 355 (P-914)9 August 1989

## Description

### BACKGROUND OF THE INVENTION

### 1. Field of the Invention:

The present invention relates to a biosensor that can easily quantify a specific component in a sample liquid with accuracy and speed, and a method for producing the same, and more particularly to a biosensor for quantifying a specific component in a sample liquid by reducing electron acceptors using electrons generated in the reaction of the specific component in the sample liquid to enzyme that specifically reacts to the component, and then by electrochemically measuring the reduced amount of electron acceptors, and a method for producing the same.

### 2. Description of the Prior Art:

Various types of biosensors utilizing specific catalyses of enzyme have been recently developed. A saccharide biosensor will be described as an example of such biosensors as follows:

The optical rotation method, the colorimetric method, the reductimetry method and other methods using different kinds of chromatographies have been developed as methods for quantitative analysis of saccharides. However, none of these methods can provide high accuracy due to the relatively low specificity against saccharides. Additionally, the optical rotation method is easy to operate but is largely influenced by the operating temperature. Therefore, it is not appropriate for common use at home and the like.

The saccharides contained in fruit are generally assessed as saccharine degrees. A refractometer of the light refraction system is often used for quantifying the saccharine degree. This refractometer functions by utilizing change of the light refractive index caused by liquid concentration. Therefore, the refractometer of the light refraction system is influenced by all the components dissolved in the sample liquid, for example, by organic acid such as citric acid or malic acid contained in fruit juice in a large amount when a saccharide in fruit is quantified. Thus, accurate quantification by this refractometer is impossible.

A glucose sensor will now be described as an example of a biosensor used in a clinical field.

A conventional method for quantifying glucose contained in blood is to centrifuge blood taken from a patient and then to measure the thus obtained blood plasma. This method requires a lot of time as well as labor. Therefore, a sensor that can directly measure glucose in blood obtained from a patient has been desired.

A sensor similar to a test paper for urinalysis has been developed as a simple glucose sensor. This glucose sensor comprises a support in a stick shape and a holder fixed to the support. The holder includes an enzyme reacting only to glucose and a dye the color of which is changed by reacting with a production of the enzyme reaction. Blood is dropped on the support of the glucose sensor and the change of the dye after a predetermined period of time of the dropping is visually observed or optically measured, whereby the content of glucose in the blood can be measured. However, the quantifying method using this glucose sensor has low accuracy due to the interference by the colored materials in the blood.

Japanese Laid-Open Patent Publication No. 1-114747 discloses the following glucose sensor with high accuracy as a method for quantifying a specific component in a sample liquid from a living body such as blood without diluting or stirring the sample liquid:

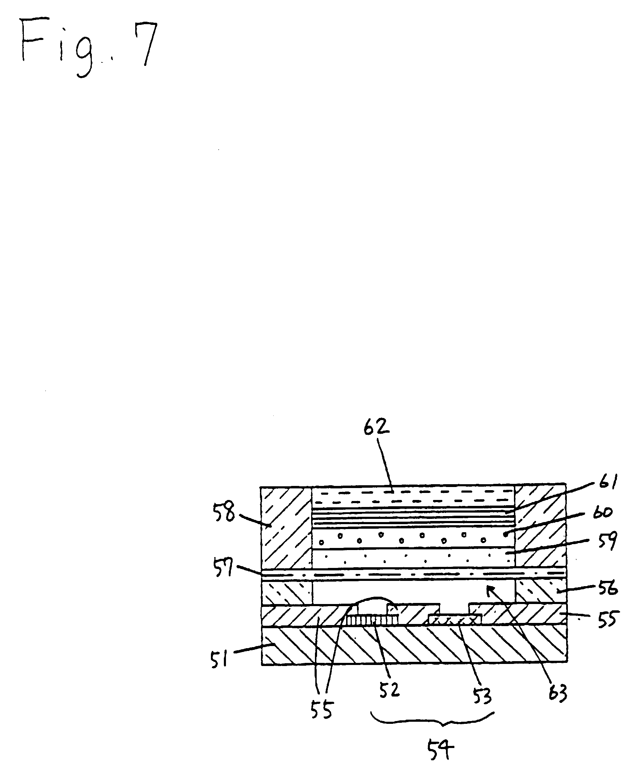

As shown in Figure **7** this glucose sensor comprises an electrical insulating substrate **51**, an electrode system **54** including a working electrode **52** and a counter electrode **53** formed on the insulating substrate **51** by screen printing, an electrical insulating layer **55** formed on the insulating substrate **51**, an adhesive structure **56** provided on the electrical insulating layer **55**, a filtration layer **57** supported by the adhesive structure **56**, a holding frame **58** provided on the filtration layer **57**, and an electron acceptor holding layer **59**, an enzyme holding layer **60**, a buffering salt holding layer **61** and an expansion layer **62** which are supported by the holding frame **58**. A space **63** for containing a sample liquid is formed between the electrode system **54** and the filtration layer **57**.

The filtration layer **57** is made from a porous polycarbonate film. The electron acceptor holding layer **59**, the enzyme holding layer **60** and the buffering salt holding layer **61** use porous cellulose as supports.

The operation of such a glucose sensor is as follows: The sample liquid dropped on the expansion layer **62** is adjusted to pH that provides the most stable enzyme activity by the action of the buffering salt in the buffering salt holding layer **61**. Then, in the enzyme holding layer **60**, the glucose oxidase in the enzyme holding layer **60** and the glucose in the sample liquid specifically react to each other. The potassium ferricyanide in the electron acceptor holding layer **59** is then reduced to become potassium ferrocyanide by electrons generated in the above reaction. The amount of the potassium ferrocyanide generated at this time is in proportion to the glucose concentration in the sample liquid. Next, materials with larger molecular weight such as protein are filtered in the filtration layer **57**, and the filtered liquid drops in the space **63** above the electrode system **54**. Thus the amount of potassium ferrocyanide in the liquid can be measured by measuring the oxidation current of the liquid by the electrode system **54**, thereby measuring the glucose concentration.

In the conventional glucose sensor with the above-mentioned structure, frothing may remain in the space due to the inconstant flow of the liquid in the space **63**, which influences the measured value of the glucose concentration.

Furthermore, since the buffering salt holding layer **61** is in contact with the enzyme holding layer 60, the buffering salt and the enzyme are mixed on the interface between the two layers when the glucose sensor absorbed moisture, thereby deteriorating the enzyme activity by the chemical interaction. As a result, the glucose sensor of this type is hard to store in a stable condition.

Moreover, since insoluble porous materials are used as supports of the filtration layer **57** and each of the holding layers **59**, **60**, and **61** in the conventional glucose sensor, the sample liquid supplied to the glucose sensor is required to pass through each of the porous materials before reaching the electrode system **54**. Therefore, the sensor has disadvantages that it may take longer time to obtain the reaction and/or that the response values may be inconstant due to the inconstant reaction time. Additionally, the glucose sensor has so many steps in its production, including such a complicated step as to assembly a plurality of porous materials, that it is difficult to produce it at a low cost.

EP-A- 0 359 831 discloses a biosensor comprising an insulating base board having formed thereon, in sequence, leads, an electrode system mainly made of carbon, an insulating layer and a reaction layer composed of an enzyme and an electron acceptor, eventually comprising a hydrophilic high molecular substance such as starch, carboxymethyl cellulose, gelatin, acrylate, vinyl alcohol, vinylpyrrolidone and maleic anhydride. No measures are take to adjust pH of the sample solution. Especially, this sensor does not comprise any buffering system.

### SUMMARY OF THE INVENTION

The biosensor of this invention for quantifying a substrate contained in a sample liquid by reducing electron acceptors using electrons generated in a reaction of the substrate to enzyme and then by measuring the reduced amount of the electron acceptors electrochemically, which overcomes the above-discussed and numerous other disadvantages and deficiencies of the prior art, comprises an electrically insulating substrate, an electrode system including at least a working electrode and a counter electrode which are formed on the insulating substrate, a reaction layer provided on the electrode system and including the enzyme and a hydrophilic polymer, the enzyme and the polymer being present in a mixed layer or splitted up into two layers, and a hydrogen ion concentration control layer, wherein the reaction layer is in contact with the electrode system and the electron acceptor is comprised in the reaction layer or in the hydrogen ion concentration control layer, and is characterized in that the hydrogen ion concentration control layer is separated from the reaction layer.

In a preferred embodiment the hydrogen ion concentration control layer is separated spatially from the reaction layer.

In a preferred embodiment the hydrogen ion concentration control layer is separated from the reaction layer by a layer of a second hydrophilic polymer.

In a preferred embodiment the reaction layer also includes a layer of a second hydrophilic polymer and electron acceptors.

In a preferred embodiment the reaction layer is formed by laminating a first layer including a hydrophilic polymer and enzyme, a second layer including a second hydrophilic polymer and a third layer including electron acceptors in this order.

In a preferred embodiment the hydrogen ion concentration control layer is provided on the electrode system, the reaction layer which further includes hydrophilic polymer and electron acceptors is provided on top of the hydrogen ion concentration control layer and separated from it by a hydrophilic polymer layer which is provided between the reaction layer and the hydrogen ion concentration control layer.

In a preferred embodiment, the electrode system is mainly formed from carbon.

In a preferred embodiment, the hydrogen ion concentration control layer includes buffering salt selected from a group consisting of potassium biphosphate-dipotassium phosphate, potassium biphosphate-disodium phosphate, citric acid-disodium phosphate, citric acid-trisodium citrate, potassium bicitrate-sodium hydroxide and maleic acid monosodium salt-sodium hydroxide.

In a preferred embodiment, the hydrophilic polymer is selected from a group consisting of polyvinyl alcohol and cellulose derivatives, concretely, hydroxy proryl cellulose, methyl cellulose, ethyl cellulose, ethyl hydroxyethyl cellulose and carboxy methyl ethyl cellulose, polyvinylpyrrolidone, gelatin or its derivatives, (meth)acrylic acid or its salts, starch or its derivatives and maleic anhydride or its salts.

In a preferred embodiment, the enzyme is selected from a group consisting of fructose dehydrogenase, invertase, mutarotase, glucose oxidase, alcohol oxidase, lactase oxidase, lactase dehydrogenase, cholesterol oxidase, xanthine oxidase and amino acid oxidase.

In a preferred embodiment, the electron acceptor is selected from a group consisting of potassium ferricyanide, p-benzoquinone, phenazinemethosulfate and ferrocene.

Alternately the present invention provides a method for producing a biosensor for quantifying a substrate contained in a sample liquid by reducing electron acceptors using electrons generated in a reaction of the substrate to enzyme and then by measuring the reduced amount of the electron acceptors eletrochemically, the method comprising the steps of: firstly, providing an electrode system including at least a working electrode and a counter electrode on an electrical insulating substrate; secondly, forming a hydrogen ion concentration control layer including electron acceptors on the insulating substrate; and finally, forming a reaction layer including hydrophilic polymer and enzyme on the electrode system, the polymer and the enzyme being in same layer of separate layers, characterized in tha the hydrogen ion concentration control layer is formed so as to be separate from the reaction layer.

Thus, the invention described herein makes possible the objectives of providing (1) a biosensor in which the hydrogen ion concentration of a sample liquid can be made most adequate in accordance with the type of enzyme contained in a reaction layer without prior adjustment of the hydrogen ion concentration in the sample liquid, (2) a biosensor which can easily quantify a specific substrate contained in a sample liquid with accuracy and speed, (3) a biosensor in which the reliability in performance thereof while being stored can be improved by separating buffering salt contained in a hydrogen ion concentration control layer from enzyme contained in a reaction layer by a hydrophilic polymer layer, (4) a method for producing a biosensor in a short period of time by heating layers such as a hydrogen ion concentration control layer including electron acceptors, and (5) a method for producing a biosensor, in which crystal diameters of electron acceptors contained in a layer can be optionally controlled by controlling the temperature in heating the layer.

### BRIEF DESCRIPTION OF THE DRAWINGS

This invention may be better understood and its numerous objects and advantages will become apparent to those skilled in the art by reference to the accompanying drawings as follows:
Figure **1** is a sectional view of a base of a fructose sensor according to an example of a biosensor of the present invention;
Figures **2** and **3** are exploded perspective views of the fructose sensor of Figure **1**;
Figure **4** is a diagram showing the response characteristics of the fructose sensor;
Figure **5** is a sectional view of a base of a fructose sensor according to another example of the present invention;
Figure **6** is an exploded perspective view of the fructose sensor of Figure **5**; and
Figure **7** is a diagram showing an example of a conventional biosensor.

### DESCRIPTION OF THE PREFERRED EMBODIMENTS

A biosensor according to the present invention comprises an electrical insulating substrate, an electrode system including a working electrode and a counter electrode provided on the insulating substrate, a reaction layer including enzyme provided on the electrode system and a hydrogen ion concentration control layer separated from the reaction layer.

According to the present invention, the enzyme is not influenced by the buffering salt included in the hydrogen ion concentration control layer because the reaction layer including the enzyme is formed separately from the hydrogen ion concentration control layer. Therefore, the contained enzyme can be kept in a stable condition when the biosensor is stored.

Generally, the pH of a sample liquid is not necessarily the same as that of the specified enzyme in the reaction layer that provides the highest enzyme activity. In accordance with the present invention, the pH of the sample liquid can approximate to the value which provides the highest activity of the enzyme by allowing the sample liquid supplied to the sensor to reach the hydrogen ion concentration control layer. As a result, the sensor can be simply operated because there is no need to adjust the pH of the sample liquid by a buffer solution or the like.

A method for producing a biosensor of the present invention comprises steps of providing an electrode system including at least a working electrode and a counter electrode on an electrical insulating substrate, forming a hydrogen ion concentration control layer including at least electron acceptors, and finally forming a reaction layer including at least hydrophilic polymer and enzyme on the electrode system, but separate from the hydrogen ion concentration control layer.

Generally the activity of enzyme is largely reduced in a treatment at a temperature of several tens degrees centigrade. According to the method of the present invention, the reaction layer is not treated at a high temperature that may inhibit the enzyme activity, since the hydrogen ion concentration control layer including the electron acceptors is formed prior to the formation of the reaction layer including the enzyme. Therefore, it is possible to set the heat treatment condition of the hydrogen ion concentration control layer optionally depending upon the objectives of the biosensors. For example, the particle diameter of the crystal of the electron acceptor or the drying condition thereof can be controlled so as to be most appropriate, by making shorter the heating time of the hydrogen ion concentration control layer and/or by controlling the heating temperature of the hydrogen ion concentration control layer.

The use of the biosensor of the present invention depends upon the substrate (the specified component), that is, the object to be measured, contained in the sample liquid. The sensor may be used as, for example, a fructose sensor, a sucrose sensor, a glucose sensor, an alcohol sensor, a lactic acid sensor, a cholesterol sensor and an amino acid sensor.

The enzyme used in the present invention depends upon the substrate contained in the sample liquid and is not limited. The usable enzyme are, for example, fructose dehydrogenase, invertase, mutarotase, glucose oxidase, alcohol oxidase, lactase oxidase, lactase dehydrogenase, cholesterol oxidase, xanthine oxidase and amino acid oxidase.

Any buffer solution with pH that provides the highest activity of enzyme used for producing a biosensor may be chosen in order to form the hydrogen ion concentration control layer. The usable buffer solutions are, for example, a phosphate buffer solution, a McIlvaine buffer solution, citric acid-trisodium citrate, potassium bicitrate-sodium hydroxide and maleic acid monosodium salt-sodium hydroxide. Further, a buffer solution which is liquid at an ordinary temperature such as an acetic acid-sodium acetate buffer solution may be used. In this case, the hydrogen ion concentration control layer may be formed together with a polymer with a long shelf life.

The hydrophilic polymer used in this invention is not limited. The usable hydrophilic polymers are, for example, polyvinyl alcohol and cellulose derivatives, concretely, hydroxy proryl cellulose, methyl cellulose, ethyl cellulose, ethyl hydroxyethyl cellulose and carboxy methyl ethyl cellulose, polyvinylpyrrolidone, gelatin or its derivatives, (meth)acrylic acid or its salts, starch or its derivatives and maleic anhydride or its salts.

As for the electron acceptors in this invention, potassium ferricyanide, p-benzoquinone, phenazinemethosulfate and ferrocene may be used.

The above-described electrode system is not limited to a two-electrode system having only a working electrode and a counter electrode. For example, a three-electrode system, including an additional reference electrode, may be used, so that more precise values are obtainable.

### EXAMPLES

Throughout the drawings mentioned in the following description of the examples, the same element has a common reference numeral. Part of the description of the producing procedure is omitted as occasion demands.

### Example 1

A fructose sensor will now be described as an example of a biosensor.

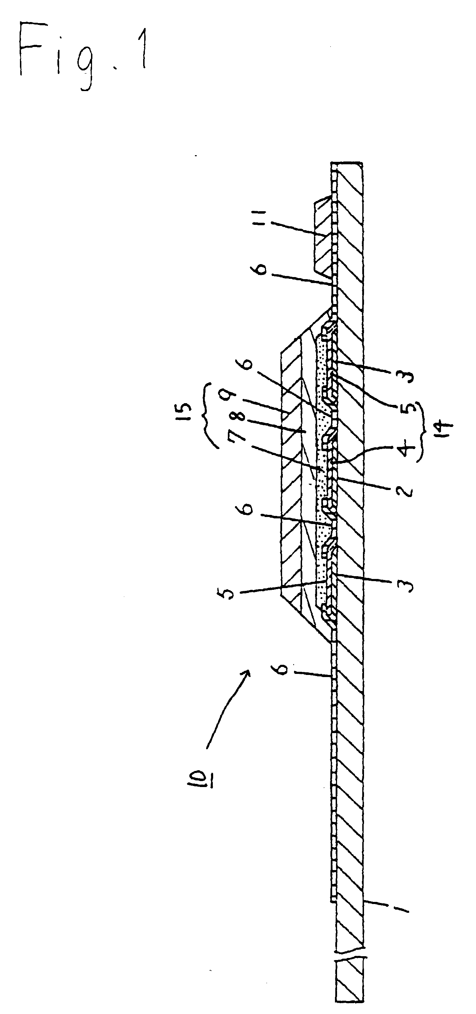

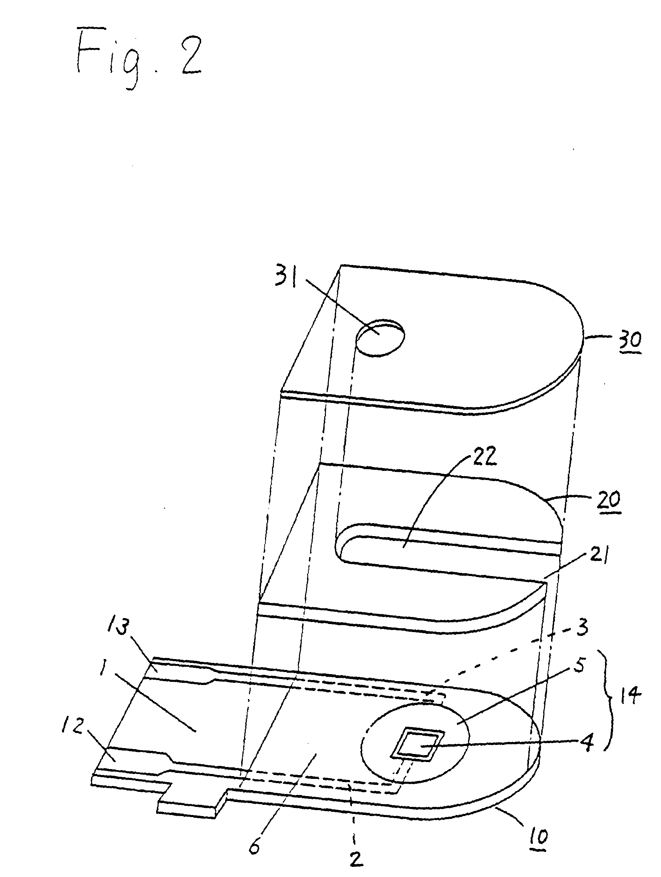

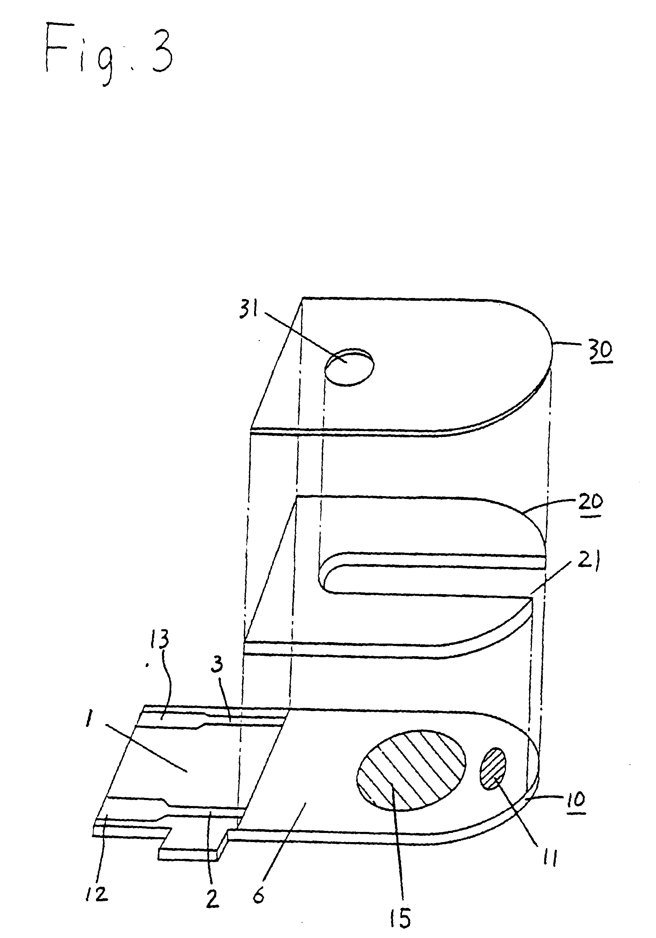

A fructose sensor of the present invention comprises a base **10**, a spacer **20** adhered to the base **10** and a cover **30** adhered to the spacer **20** as shown in Figures **1** to **3**. Figure **1** shows a sectional view of the base **10** of the fructose sensor, and Figure **2** shows an exploded perspective view of the fructose sensor with a reaction layer **15** and a hydrogen ion concentration control layer **11** removed from the base **10**. Figure **3** is also an exploded perspective view of the fructose sensor.

Silver paste was printed by means of screen printing on an electrical insulating substrate **1** made from polyethylene terephthalate to form leads **2** and **3**. Then, an electrode system **14** (a working electrode **4** and a counter electrode **5**) made from conductive carbon paste including resin binder and an insulating layer **6** made from insulating paste were formed by screen printing. The insulating layer **6** was covering the leads **2** and **3** except for connecting portions **12** and **13** with a connector so that the predetermined areas of the working electrode **4** and the counter electrode **5** were exposed respectively.

Then, the exposed portions of the working electrode **4** and the counter electrode **5** were ground and heat treated for 4 hours at 100°C in the air.

After forming the electrode system **14** comprising the working electrode **4** and the counter electrode **5** in this way, an aqueous solution including 0.5 wt% of carboxymethyl cellulose (hereinafter called the CMC), as hydrophilic polymer, was dropped on the electrode system **14** and dried, to form a CMC layer. Then an aqueous solution of fructose dehydrogenase (manufactured by Toyo Boseki Kabushiki Kaisha; EC1.1.99.11), as enzyme, was dropped so as to cover the CMC layer and dried, to form a CMC-fructose dehydrogenase layer **7**. In this CMC-fructose dehydrogenase layer **7**, the CMC layer and the fructose dehydrogenase layer were mixed only by a thickness of several microns on the interface therebetween, namely, both layers were not completely mixed.

An ethanol solution including 0.5 wt% of polyvinylpyrrolidone (hereinafter called the PVP), as hydrophilic polymer, was dropped so as to completely cover the CMC-fructose dehydrogenase layer **7** and dried, to form a PVP layer **8**.

When fruit juice including solid elements such as sarcocarp is used as a sample liquid, the solid elements are stopped by the PVP layer **8** provided on the CMC-fructose dehydrogenase layer **7**, resulting in the prevention of the solid elements from being adsorbed to the surface of the electrode system **14**. As a result, the response function of the sensor is prevented from being deteriorated. Furthermore, since the PVP layer **8** separates the fructose dehydrogenase contained in the CMC-fructose dehydrogenase layer **7** from potassium ferricyanide described below, the conservative and stable properties of the sensor can be remarkably improved.

Dispersing liquid, with crystals of potassium ferricyanide (the particle diameter: 0.5 - 4 µm), that is, electron acceptors, dispersed in a toluene solution including 0.5 wt% of lecithin as a dispersing agent was dropped and dried at room temperature, to form a potassium ferricyanide-lecithin layer **9** on the PVP layer **8**.

Thus the reaction layer **15** comprising the CMC-fructose dehydrogenase layer **7**, the PVP layer **8** and the potassium ferricyanide-lecithin layer **9** was formed.

Then, 3 µl of a phosphate buffer solution (a mixture of 0.2 M of K₂HPO₄ and 0.2 M of KH₂PO₄, pH = 4.5) was dropped on a portion between the reaction layer **15** and the tip portion of the insulating substrate corresponding to a sample supply port **21**, and dried, to form a hydrogen ion concentration control layer **11** as shown in Figures **1** and **3**.

The enzyme used in this fructose sensor, that is, fructose dehydrogenase, shows the highest activity when pH is 4.5 (at 37°C). Since a fructose standard solution is almost neutral, when the standard solution reaches the hydrogen ion concentration control layer **11**, the pH of the solution becomes 4.5, thereby making the enzyme activity the highest. Alternately, when the sample liquid is acid or alkaline, the pH of the liquid is adjusted to be approximately 4.5, when the liquid reaches the hydrogen ion concentration control layer **11**. Therefore, in the liquid that has passed the hydrogen ion concentration control layer **11**, the enzyme activity will become higher as compared with in the originally supplied sample liquid.

Furthermore, the enzyme in the reaction layer **15** is separated from the hydrogen ion concentration control layer **11** by forming the hydrogen ion concentration control layer **11** away from the reaction layer **15**, thereby the preservative property of the sensor is improved.

After forming the base **10** including the reaction layer **15** and the hydrogen ion concentration control layer **11** in the above-described way, the base **10**, the spacer **20** and the cover **30** were laminated to be adhered as shown in Figures **2** and **3** with dashed lines. A reservoir for a sample liquid was formed as a space defined by the base **10**, the wall of a groove **22** of the spacer **20** and the cover **30**. As a result the sample liquid supply port **21** was formed at one end of the reservoir. An air port **31** was formed on a portion of the cover **30** opposing to the other end of the reservoir.

A sample liquid is supplied into the reservoir and introduced toward the reaction layer **15** only by allowing the sample liquid to contact the sample supply port **21** formed at the tip of the sensor. Since the supply amount of the sample liquid depends upon the volume of the reservoir, prior quantification of the liquid is not necessary. Furthermore, evaporation of the sample liquid during the measurement can be minimized, resulting in a measurement with high accuracy.

The thus produced fructose sensor was supplied with 3 µl of a fructose standard solution as a sample liquid from the sample supply port **21**. Two minutes after the supply, a pulse voltage of +0.5 V on the basis of the voltage at the counter electrode **5** was applied to the working electrode **4**. Then the anodic current value of five seconds after the application was measured.

When the sample liquid having obtained the desired pH after passing the hydrogen ion concentration control layer **11** reaches the reaction layer **15**, the potassium ferricyanide-lecithin layer **9**, the PVP layer **8** and the CMC-fructose dehydrogenase layer **7** are dissolved into the sample liquid successively in this order. The fructose contained in the sample liquid is oxidized by fructose dehydrogenase, and at this point the potassium ferricyanide is reduced to potassium ferrocyanide by shifted electrons. Then, an oxidation current corresponding to the concentration of the generated potassium ferrocyanide is caused by the application of the above pulse voltage. The current value corresponds to the concentration of the substrate, that is, fructose in this case.

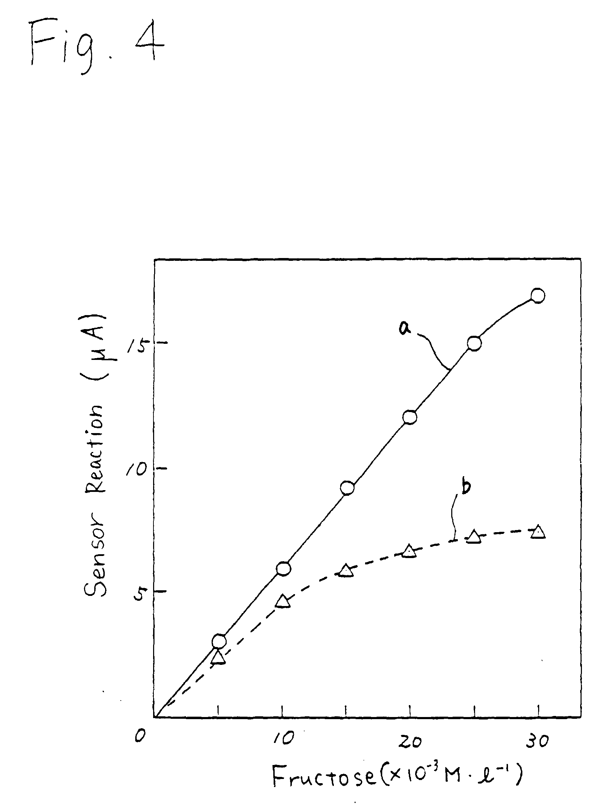

Figure **4** shows the response property measured two minutes after the supply of the sample liquid by using the fructose sensor of this example. In the graph, the curve a shows responses of the fructose sensor of this example. The curve **b** shows responses of a comparative fructose sensor, in which the hydrogen ion concentration control layer **11** is removed from the base **10** of the fructose sensor, obtained in the same way as in this example.

As is evident from Figure **4**, in the fructose sensor of this example, the relation between the fructose concentration and the sensor response corresponded proportionally up to 25 mM of fructose, the substrate. On the other hand, in the fructose sensor without a hydrogen ion concentration control layer, the fructose concentration corresponded to the sensor response only up to 10 mM of fructose.

In Figure **4**, in the range where the corresponding relation between the fructose concentration and the sensor response is shown as a straight line, the response current value obtained by the fructose sensor of this example was larger by about 20% than that of the comparative fructose sensor without the hydrogen ion concentration control layer **11**.

This example shows that a biosensor with a wide measuring range and a high sensitivity can be obtained by providing the hydrogen ion concentration control layer **11** on the base **10**.

Furthermore, in the fructose sensor of this example, when the measuring time was set to be one minute, the relation that can be shown as a straight line was obtained up to 15mM of fructose. The response repeatability of the sensor was also excellent. For example, the coefficient of variation (the CV value) in using thirty sensors as to the same sample liquid was 7% or less.

### Example 2

An electrode system **14** comprising a working electrode **4** and a counter electrode **5** as shown in Figure **1** was formed on an insulating substrate **1** made from polyethylene terephthalate by means of screen printing in the same manner as in Example 1, and was ground and heat treated. A mixed aqueous solution including 0.5 wt% of CMC, fructose dehydrogenase and potassium ferricyanide was dropped on the electrode system **14** and was dried for 10 minutes in a warm-air drier at 40°C, to form a reaction layer **15**.

The production procedure of a biosensor can be simplified by forming the reaction layer **15** by dropping and drying a mixed solution of hydrophilic polymer, enzyme and electron acceptors on the electrode system **14** in the above way.

Then, 3 µl of a McIlvaine buffer solution (a mixture of 0.1 M of citric acid and 0.2 M of disodium phosphate, pH = 4.5) was dropped on a portion between the reaction layer **15** and a tip portion of the insulating substrate **1** corresponding to a sample supply port **21** of the sensor, and was dried to form a hydrogen ion concentration control layer **11**. The thus obtained base **10**, a spacer **20** and a cover **30** were laminated to be adhered as in Example 1 as shown in Figures **2** and **3** with dashed lines.

Three µl of a fructose standard solution was supplied to the thus obtained fructose sensor from the sample supply port **21**. Two minutes after the supply, a pulse voltage of +0.5 V on the basis of the voltage of the counter electrode **5** was applied to the working electrode **4**. The anodic current value 5 seconds after the application was measured, resulting in obtaining a response current value corresponding to the fructose concentration in the sample liquid. Alternately, the sample liquid was supplied in the same manner and the voltage was applied after 90 seconds. The obtained response current was almost the same as that obtained after 2 minutes. Moreover, the coefficient of variation measured in using 30 sensors as to the same sample liquid was 5% or less. Thus, the repeatability was also excellent.

In the fructose sensor of this example, the fructose dehydrogenase mixed with potassium ferricyanide exists in the reaction layer **15**. Therefore, both the fructose dehydrogenase and the potassium ferricyanide are easily dissolved into the sample liquid rapidly and uniformly. This rapid reaction in the mixture reduces the time required for measuring and makes the response current constant.

The response current obtained by the fructose sensor of this example was larger by about 20% than that of the comparative fructose sensor without a hydrogen ion concentration control layer as was used in Example 1.

### Example 3

An electrode system **14** comprising a working electrode **4** and a counter electrode **5** as shown in Figure **1** was formed on an insulating substrate **1** made from polyethylene terephthalate by means of screen printing in the same manner as in Example 1, and was ground and heat treated. Then, 3 µl of a phosphate buffer solution (a mixture of 0.2 M of K₂HPO₄ and 0.2 M of KH₂PO₄, pH = 4.5) including potassium ferricyanide was dropped on a portion between the electrode system **14** and the tip portion of the insulating substrate corresponding to a sample supply port **21**, and was dried at 100°C for 5 minutes by heating as shown in Figures **1** and **3**, to form a hydrogen ion concentration control layer **11** including potassium ferricyanide.

The time required for drying the hydrogen ion concentration control layer **11** depends upon the temperature at which the layer is heated. Therefore, the crystal diameter of the potassium ferricyanide contained in the hydrogen ion concentration control layer **11** can be controlled by the heat treatment condition. The crystal diameter of the potassium ferricyanide becomes smaller when the drying time is shorter, resulting in increasing the solubility speed into the sample liquid. Thus the response speed of the sensor can be increased. On the other hand, when the potassium ferricyanide coexists with the enzyme in the reaction layer **15**, the enzyme activity is reduced by heating the reaction layer **15** at a high temperature. Therefore, the heating temperature of the layer **15** can not be set freely.

Furthermore, as in the present example, when the enzyme in the reaction layer **15** is separated from the potassium ferricyanide by containing the potassium ferricyanide in the hydrogen ion concentration control layer **11**, the shelf life of the sensor can be remarkably increased.

Then, a mixed aqueous solution including 0.5 wt% of CMC and fructose dehydrogenase was dropped on the electrode system **14**, and was dried in a warm-air drier at 40°C for 10 minutes, to form the reaction layer **15**. The thus formed base **10**, a spacer **20** and a cover **30** were laminated to form a fructose sensor.

The fructose sensor produced in the above way was supplied with 3 µl of a fructose standard solution from the sample supply port **21**. The sensor response was measured 2 minutes after the supply in the same manner as in Example 1. As a result, the obtained current value corresponded to the fructose concentration in the sample liquid. Alternately, when the voltage was applied 90 seconds after supplying the sample liquid as described above, the response current value was approximately the same as that obtained after 2 minutes. This is because the potassium ferricyanide in the sample liquid was rapidly dissolved due to the smaller crystal diameters of the potassium ferricyanide (0.05 - 0.3 µm).

The response current obtained by the fructose sensor of this example was larger by about 20% than that of the comparative fructose sensor without a hydrogen ion concentration control layer **11** as was in Example 1.

### Example 4

A sucrose sensor will now be described as another example of a biosensor.

An electrode system **14** comprising a working electrode **4** and a counter electrode **5** as shown in Figure **1** was formed on an insulating substrate **1** made from polyethylene terephthalate, and was ground and heat treated. Then, an aqueous solution including 0.5 wt% of CMC was dropped on the electrode system **14** and was dried to form a CMC layer. Then a mixed aqueous solution including 0.5 wt% of CMC, invertase (EC 3.2.1.26), mutarotase (EC5.1.3.3), glucose oxidase (EC1.1.3.4) and potassium ferricyanide was dropped on the CMC layer and was dried in a warm-air drier at 40°C for 10 minutes to form a reaction layer **15**.

Then a phosphate buffer solution (pH = 7.4) was dropped on a portion between the reaction layer **15** and the tip portion of the insulating substrate **1** corresponding to a sample supply port **21**, then was dried to form a hydrogen ion concentration control layer **11**. The thus obtained base **10**, a spacer **20** and a cover **30** were laminated to form a sucrose sensor.

Three µl of a sucrose standard solution was supplied to the sucrose sensor from the sample supply port **21**. Two minutes after the supply, a pulse voltage of +0.5 V on the basis of the voltage of the counter electrode **5** was applied to the working electrode **4**. Then the anodic current value after 5 seconds was measured. As a result, the obtained response current value corresponded to the sucrose concentration in the sample liquid.

### Example 5

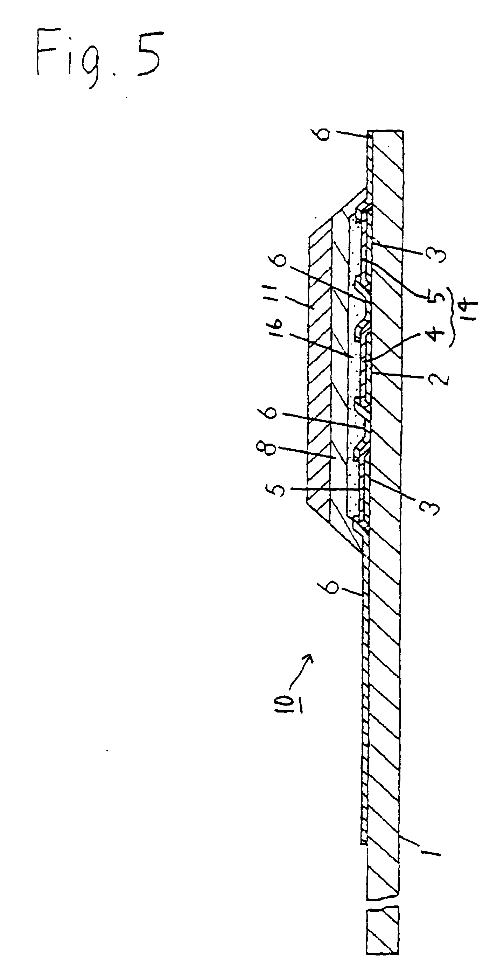

An electrode system **14** comprising a working electrode **4** and a counter electrode **5** was formed on an insulating substrate **1** made from polyethylene terephthalate by means of screen printing as shown in Figure **5**, and was ground and heat treated. A mixed aqueous solution including 0.5 wt% of CMC, fructose dehydrogenase and potassium ferricyanide was dropped on the electrode system **14**, and was dried in a warm-air drier at 40°C for 10 minutes to form a CMC-fructose dehydrogenase-potassium ferricyanide layer **16**.

Then, an ethanol solution including 0.5 wt% of PVP as hydrophilic polymer was dropped so as to cover the entire CMC-fructose dehydrogenase-potassium ferricyanide layer **16**, and was dried to form a PVP layer **8**. 3 µl of a McIlvaine buffer solution (a mixture of 0.1 M of citric acid and 0.2 M of disodium phosphate, pH = 4.5) was dropped on the PVP layer **8**, and was dried to form a hydrogen ion concentration control layer **11**.

In this example, the hydrogen ion concentration control layer **11** and the PVP layer **8** are mixed only by a thickness of several microns on the interface therebetween since PVP is hydrophilic polymer. Therefore, the CMC-fructose dehydrogenase-potassium ferricyanide layer **16** is separated from the hydrogen ion concentration control layer **11** by the PVP layer **8**. In this example, a reaction layer **15** comprises the CMC-fructose dehydrogenase-potassium ferricyanide layer **16**.

Thus, the time required for solution of the reaction layer **15** into the sample liquid after the hydrogen ion concentration control layer **11** is dissolved, because the hydrogen ion concentration control layer **11** is formed on the reaction layer **15**, thereby reducing the time required for the sensor response.

Furthermore, in the fructose dehydrogenase used in this example as enzyme, the pH for allowing the enzyme activity to be the highest is different from the pH for providing a high conservative stability of the sensor. Therefore, in the conventional biosensor as shown in Figure **7**, the shelf life of the sensor is deteriorated because the sensor is under a pH condition which is different from that providing the fructose dehydrogenase with a high stability when the biosensor absorbs moisture.

The biosensor of this example can overcome the above-mentioned disadvantage because the CMC-fructose dehydrogenase-potassium ferricyanide layer **16** can be separated from the hydrogen ion concentration control layer **11** by the PVP layer **8**.

Moreover, in the biosensor of this example, it is not necessary to distinguish a sample supply port **21** provided to the sensor from an air port **31**. Therefore, the sample supply port **21** can be used as an air port, from which it is possible to supply the sample liquid.

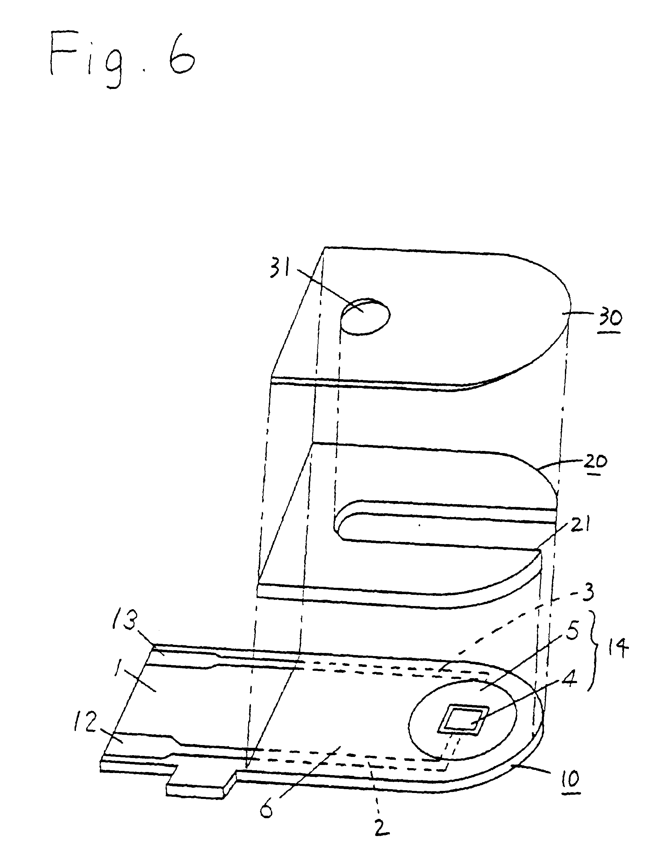

After forming the base **10** including the reaction layer **15** and the hydrogen ion concentration control layer **11** in the above-mentioned manner, the base **10**, a spacer **20** and a cover **30** were laminated so as to be adhered as shown in Figure **6** with dashed lines.

The thus obtained fructose sensor was supplied with 3 µl of a fructose standard solution as a sample liquid from the sample supply port **21**, and the sensor response was measured in the same manner as in Example 1. The obtained result was a proportional linear relation up to 25 mM of fructose. Furthermore, the coefficient of variation (the CV value) obtained in using 30 sensors was 6% or less. Thus the repeatability was excellent.

### Example 6

An electrode system **14** comprising a working electrode **4** and counter electrode **5** was formed on an insulating substrate **1** made from polyethylene terephthalate by means of screen printing in the same manner as in Example 1, and was ground and heat treated. An aqueous solution including potassium ferricyanide and CMC was dropped on the electrode system **14** and dried by heating, to form a layer including CMC and potassium ferricyanide.

The time required for drying the layer depends upon the heating temperature. Therefore, the crystal diameter of the potassium ferricyanide contained in the layer can be controlled by the heat treatment condition. A shorter drying time makes the crystal diameter of the potassium ferricyanide smaller, therefore increasing solubility speed into the sample liquid. In this way, the response speed can be increased. On the other hand, when potassium ferricyanide coexists with enzyme in the reaction layer, the heating temperature can not be freely set, because the enzyme activity is reduced by heating at a high temperature.

Then, an ethanol solution including 0.5 wt% of PVP was dropped so as to cover the entire layer including potassium ferricyanide and CMC, and was dried to form a PVP layer. Furthermore, a fructose dehydrogenase solution was dropped on the PVP layer, and was dried to form a fructose dehydrogenase layer.

Then, an ethanol solution including 0.5 wt% of hydroxyethyl cellulose so as to cover the entire fructose dehydrogenase layer, and was dried to form a hydroxyethyl cellulose layer. Furthermore, a phosphate buffer solution (pH = 4.5) was dropped on the hydroxyethyl cellulose layer, and was dried to form a hydrogen ion concentration control layer **11**. The thus obtained base **10**, a spacer **20** and a cover **30** were laminated in the same manner as in Example 1 to form a fructose sensor.

PVP and hydroxyethyl cellulose are hydrophilic polymer. Therefore, when an aqueous solution including fructose dehydrogenase is dropped on the PVP layer, the PVP layer and the fructose dehydrogenase layer are not completely mixed but only mixed by a thickness of several microns on the interface therebetween. Also when a phosphate buffer solution is dropped on the hydroxyethyl cellulose layer, the hydroxyethyl cellulose layer and the hydrogen ion concentration control layer are not completely mixed but only mixed by a thickness of several microns on the interface therebetween.

In this way, the CMC-potassium ferricyanide layer is separated from the fructose dehydrogenase layer and the hydrogen ion concentration control layer respectively.

The fructose sensor produced in the above method was supplied with 3 µl of a fructose standard solution from a sample supply port. The sensor response after 2 minutes obtained in the same manner as in Example 1 corresponded to the fructose concentration in the sample liquid. Alternately, when the voltage was applied 90 seconds after the supply, the response current was approximately the same as that obtained 2 minutes after. This is because potassium ferricyanide dissolved into the sample liquid more quickly due to the smaller crystal diameter of the potassium ferricyanide.

The response current values obtained by the fructose sensor of this example were larger by about 20% than those obtained by the comparative fructose sensor without a hydrogen ion concentration control layer **11**, as was in Example 1.

## Claims

1. A biosensor for quantifying a substrate contained in a sample liquid by reducing electron acceptors using electrons generated in a reaction of the substrate to enzyme and then by measuring the reduced amount of the electron acceptors electrochemically, the biosensor comprising:
- an electrically insulating substrate (1), an electrode system (14) including at least a working electrode (4) and a counter electrode (5) which are formed on the insulating substrate (1),
- a reaction layer (15) provided on the electrode system (14) and including the enzyme and a hydrophilic polymer, the enzyme and the polymer being present in a mixed layer or splitted up into two layers , and
- a hydrogen ion concentration control layer (11);
- wherein the reaction layer (15)is in contact with the electrode system (14) and the electron acceptor is comprised in the reaction layer or in the hydrogen ion concentration control layer,
characterized in that
- the hydrogen ion concentration control layer (11) is separated from the reaction layer (15).

2. A biosensor according to claim 1, wherein the hydrogen ion concentration control layer (11) is separated spatially from the reaction layer (15).

3. A biosensor according to claim 1, wherein the hydrogen ion concentration control layer (11) is separated from the reaction layer (15) by a layer of a second hydrophilic polymer.

4. A biosensor according to claims 1 to 3, wherein the reaction layer (15) also includes a layer of a second hydrophilic polymer and electron acceptors.

5. A biosensor according to claims 2 or 4, wherein the reaction layer (15) is formed by laminating a first layer including a hydrophilic polymer and enzyme, a second layer including a second hydrophilic polymer and a third layer including electron acceptors in this order.

6. A biosensor according to claim 3, wherein
- the hydrogen ion concentration control layer (11) is provided on the electrode system (14),
- the reaction layer (15) which further includes hydrophilic polymer and electron acceptors is provided on top of the hydrogen ion concentration control layer (11) and separated from it by a hydrophilic polymer layer which is provided between the reaction layer (15) and the hydrogen ion concentration control layer (11).

7. A biosensor according to claims 1 to 6, wherein the electrode system (14) is mainly formed from carbon.

8. A biosensor according to claims 1 to 7, wherein the hydrogen ion concentration control layer (11) includes a buffering salt selected from the group consisting of potassium biphosphate-dipotassium phosphate, potassium biphosphate-disodium phosphate, citric acid-disodium phosphate, citric acid-trisodium citrate, potassium bicitrate-sodium hydroxide and maleic acid monosodium salt-sodium hydroxide.

9. A biosensor according to claims 1 to 8, wherein the hydrophilic polymer selected from the group consisting of carboxmethyl cellulose, polyvinylpyrrolidone, hydroxy ethyl cellulose, hydrox propyl cellulose, methyl cellulose, ethyl cellulose, ethyl hydroxyethyl cellulose, carboxy methyl ethyl cellulose, polyvinyl alcohol and gelatin.

10. A biosensor according to claims 1 to 9, wherein the enzyme is selected from the group consisting of fructose dehydrogenase, invertase, mutarotase, glucose oxidase, alcohol oxidase, lactate oxidase, lactate dehydrogenase, cholesterol oxidase, xanthine oxidase and amino acid oxidase.

11. A biosensor according to claims 1 to 10, wherein the electron acceptor is selected from the group consisting of potassium ferricyanide, p-benzoquinone, phenazinemethosulfate and ferrocene.

12. A method for producing a biosensor for quantifying a substrate contained in a sample liquid by reducing electron acceptors using electrons generated in a reaction of the substrate to enzyme and then by measuring the reduced amount of the electron acceptors electrochemically, the method comprising the steps of:
- firstly, providing an electrode system (14) including at least a working electrode and a counter electrode (5) on an electrical insulating substrate (1);
- secondly, forming a hydrogen ion concentration control layer (11) including electron acceptors on the insulating substrate (1); and
- finally, forming a reaction layer (15) including hydrophilic polymer and enzyme on the electrode system (14), the polymer and the enzyme being in same layer or separate layers,
characterized in that the hydrogen ion concentration control layer (11) is formed so as to be separate from the reaction layer (15).

## Patentansprüche

1. Biosensor zur Quantifizierung eines in einer Probenflüssigkeit enthaltenen Substrats durch Reduktion eines Elektronenakzeptors mittels Elektronen, die in der Reaktion des Substrats mit einem Enzym erzeugt werden, und anschließende elektrochemische Messung der Menge an reduziertem Elektronenakzeptor, umfaßend:
- eine elektrisch isolierende Grundlage (1), ein Elektrodensystem (14), enthaltend mindestens eine Arbeitselektrode (4) und eine Gegenelektrode (5), die auf der isolierenden Grundlage (1) ausgebildet sind,
- eine Reaktionsschicht (15), die auf dem Elektrodensystem (14) ausgebildet ist und das Enzym und ein hydrophiles Polymer enthält, wobei das Enzym und das Polymer in einer Mischschicht vorliegen oder auf zwei Schichten aufgeteilt sind, und
- eine Schicht (11) zum Einstellen der Wasserstoffionenkonzentration (H-Ionen-Einstellschicht),
- wobei die Reaktionsschicht (15) mit dem Elektrodensystem (14) in Kontakt steht und der Elektronenakzeptor entweder in der Reaktionsschicht oder der H-Ionen-Einstellschicht enthalten ist,
dadurch gekennzeichnet, daß
die H-Ionen-Einstellschicht (11) von der Reaktionsschicht (15) getrennt ist.

2. Biosensor gemäß Anspruch 1, bei dem die H-Ionen-Einstellschicht (11) räumlich von der Reaktionsschicht (15) getrennt ist.

3. Biosensor gemäßß Anspruch 1, bei dem die H-Ionen-Einstellschicht (11) von der Reaktionsschicht (15) durch eine Schicht aus einem zweiten hydrophilen Polymer getrennt ist.

4. Biosensor gemäß einem der Ansprüche 1 bis 3, bei dem die Reaktionsschicht (15) zusätzlich eine Schicht aus einem zweiten hydrophilen Polymer und Elektronenakzeptoren enthält.

5. Biosensor gemäß einem der Ansprüche 2 oder 4, bei dem die Reaktionsschicht (15) gebildet wird, indem man eine erste, ein hydrophiles Polymer und Enzym enthaltende Schicht, eine zweite Schicht, enthaltend ein zweites hydrophiles Polymer, und eine die Elektronenakzeptoren enthaltende Schicht in dieser Reihenfolge laminiert.

6. Biosensor gemäß Anspruch 3, bei dem
- die H-Ionen-Einstellschicht (11) auf dem Elektrodensystem (14) vorgesehen ist,
- die Reaktionsschicht (15), die außerdem ein hydrophiles Polymer und die Elektronenakzeptoren enthält, oben auf der H-Ionen-Einstellschicht (11) angeordnet und von dieser durch eine Schicht aus hydrophilem Polymer getrennt ist, die zwischen der Reaktionsschicht (15) und der H-Ionen-Einstellschicht (11) vorgesehen ist.

7. Biosensor gemäß einem der Ansprüche 1 bis 6, bei dem die Elektrode im wesentlichen aus Kohlenstoff besteht.

8. Biosensor gemäß einem der Ansprüche 1 bis 7, bei dem die H-Ionen-Einstellschicht (11) ein Puffersalz enthält, das aus der aus Kaliumbiphosphat-Dikaliumphosphat, Kaliumbiphosphat-Dinatriumphosphat, Zitronensäure-Dinatriumphosphat, Zitronensäure-Trinatriumcitrat, Kaliumbicitrat-Natriumhydroxid und Apfelsäuremononatriumsalz-Natriumhydroxid bestehenden Gruppe ausgewählt ist.

9. Biosensor gemäß einem der Ansprüche 1 bis 8, bei dem das hydrophile Polymer aus einer Gruppe ausgewählt ist, die aus Hydroxypropylcellulose, Methylcellulose, Ethylcellulose, Ethylhydroxyethylcellulose und Carboxymethylethylcellulose, Polyvinylpyrrolidon, Gelatine und deren Derivaten, (Meth)acrylsäure und deren Salzen, Stärke oder deren Derivaten sowie Maleinsäure und deren Derivaten besteht.

10. Biosensor gemäß einem der Ansprüche 1 bis 9, bei dem das Enzym aus der aus Fruktosedehydrogenase, Invertase, Mutarotase, Glukoseoxidase, Alkoholoxidase, Lactatoxidase, Lactatdehydrogenase, Cholesteroloxidase, Xanthinoxidase und Aminosäureoxidase bestehenden Gruppe ausgewählt ist.

11. Biosensor gemäß einem der Ansprüche 1 bis 10, bei dem der Elektronenakzeptor aus der aus Kaliumferricyanid, p-Benzochinon, Phenazinmethosulfat und Ferrocen bestehenden Gruppe gewählt ist.

12. Verfahren zur Herstellung eines Biosensor zur Quantifizierung eines in einer Probenflüssigkeit enthaltenen Substrats durch Reduktion eines Elektronenakzeptors mittels Elektronen, die in einer Reaktion des Substrats mit einem Enzym erzeugt werden, und anschließende elektrochemische Messung der Menge an reduziertem Elektronenakzeptor, welches die folgenden Schritte umfaßt:
- erstens, Bereitstellen eines Elektrodensystems (14) mit mindestens einer Arbeitselektrode (4) und einer Gegenelektrode (5) auf einer elektrisch isolierenden Grundlage (1);
- zweitens, Ausbilden einer H-Ionen-Einstellschicht (11), die die Elektronenakzeptoren enthält, auf der isolierenden Grundlage (1); und
- schließlich, Bilden der das hydrophile Polymer und das Enzym enthaltenden Reaktionsschicht (15) auf dem Elektrodensystem (14), wobei das Polymer und das Enzym in derselben oder in getrennten Schichten enthalten sind,
dadurch gekennzeichnet, daß
die H-Ionen-Einstellschicht (11) getrennt von der Reaktionsschicht (15) ausgebildet wird.

## Revendications

1. Biocapteur pour quantifier un substrat contenu dans un échantillon liquide en réduisant les accepteurs d'électrons par utilisation des électrons libérés dans une réaction du substrat avec l'enzyme, puis en mesurant électrochimiquement la quantité réduite des accepteurs d'électrons, le biocapteur comprenant:
- un substrat électriquement isolant (1), un système d'électrodes (14) comprenant au moins une électrode de travail (4) et une contre-électrode (5) qui sont formées sur le substrat isolant (1),
- une couche réactionnelle (15) prévue sur le système d'électrodes (14) et comprenant l'enzyme et un polymère hydrophile, l'enzyme et le polymère étant présents en mélange dans une couche ou séparés en deux couches, et
- une couche (11) de régulation de la concentration en ions hydrogène;
- dans laquelle la couche réactionnelle (15) est en contact avec le système d'électrodes (14) et l'accepteur d'électrons est compris dans la couche réactionnelle ou dans la couche de régulation de la concentration en ions hydrogène,
caractérisé en ce que:
- la couche (11) de régulation de la concentration en ions hydrogène est séparée de la couche réactionnelle (15).

2. Biocapteur selon la revendication 1, dans lequel la couche (11) de régulation de la concentration en ions hydrogène est séparée spatialement de la couche réactionnelle (15).

3. Biocapteur selon la revendication 1, dans lequel la couche (11) de régulation de la concentration en ions hydrogène est séparée de la couche réactionnelle (15) par une couche d'un deuxième polymère hydrophile.

4. Biocapteur selon l'une des revendications 1 à 3, dans lequel la couche réactionnelle (15) comprend également une couche d'un deuxième polymère hydrophile et d'accepteurs d'électrons.

5. Biocapteur selon l'une des revendications 2 ou 4, dans lequel la couche réactionnelle (15) est formée en stratifiant une première couche comprenant un polymère hydrophile et une enzyme, une deuxième couche comprenant un deuxième polymère hydrophile et une troisième couche comprenant des accepteurs d'électrons, dans cet ordre.

6. Biocapteur selon la revendication 3, dans lequel:
- la couche (11) de régulation de la concentration en ions hydrogène est disposée sur le système d'électrodes (14);
- la couche réactionnelle (15) qui comprend de plus le polymère hydrophile et les accepteurs d'électrons est disposée au-dessus de la couche (11) de régulation de la concentration en ions hydrogène et est séparée de celle-ci par une couche polymère hydrophile qui est prévue entre la couche réactionnelle (15) et une couche (11) de régulation de la concentration en ions hydrogène.

7. Biocapteur selon les revendications 1 à 6, dans lequel le système d'électrodes (14) est principalement formé à partir du carbone.

8. Biocapteur selon les revendications 1 à 7, dans lequel la couche (11) de régulation de la concentration en ions hydrogène comprend un sel tampon choisi dans le groupe formé par le phosphate monopotassique/phosphate dipotassique, le phosphate monopotassique/le phosphate monosodique, l'acide citrique/le phosphate disodique, l'acide citrique/le citrate trisodique, le citrate monopotassique/hydroxyde de sodium et le sel monosodique d'acide maléique/hydroxyde de sodium.

9. Biocapteur selon l'une des revendications 1 à 3, dans lequel le polymère hydrophile est choisi dans le groupe formé par la carboxyméthylcellulose, la polyvinylpyrrolidone, l'hydroxyéthylcellulose, l'hydroxypropylcellulose, la méthylcellulose, l'éthylcellulose, l'éthylhydroxyéthylcellulose, la carboxyméthyléthylcellulose, les poly(alcool vinylique) et la gélatine.

10. Biocapteur selon l'une des revendications 1 à 9, dans lequel l'enzyme est choisie dans le groupe formé par la fructose déshydrogénase, l'invertase, la mutarotase, la glucose oxydase, l'alcool oxydase, la lactate oxydase, la lactate déshydrogénase, la cholestérol oxydase, la xanthine oxydase et l'aminoacide oxydase.

11. Biocapteur selon l'une des revendications 1 à 10, dans lequel l'accepteur d'électrons est choisi dans le groupe formé par le ferricyanure de potassium, la p-benzoquinone, le phénazineméthosulfate et le ferrocène.

12. Procédé pour produire un biocapteur en vue de quantifier un substrat contenu dans un échantillon liquide en réduisant les accepteurs d'électrons par utilisation des électrons engendrés dans une réaction du substrat avec l'enzyme, puis en mesurant électrochimiquement la quantité réduite des accepteurs d'électrons, le procédé comprenant les étapes consistant:
- premièrement, à fournir un système d'électrodes (14) comprenant au moins une électrode de travail et une contre-électrode (5) sur un substrat électriquement isolant (1);
- deuxièmement, à former une couche (11) de régulation de la concentration en ions hydrogène comprenant des accepteurs d'électrons sur le substrat isolant (1); et
- finalement, à former une couche réactionnelle (15) comprenant un polymère hydrophile et l'enzyme sur le système d'électrodes (14), le polymère et l'enzyme étant dans la même couche ou dans des couches séparées;
caractérisé en ce que la couche (11) de régulation de la concentration en ions hydrogène est formée de façon à être séparée de la couche réactionnelle (15).
